# EUROPEAN PATENT APPLICATION

(11) **EP 4 190 254 A1**
(43) Date of publication of application: **07.06.2023**
(21) Application number: 21212217.0
(22) Date of filing: 03.12.2021
(51) Int. Cl.: A61B 17/64, A61B 17/28

(54) **TOOL**

(71) Applicant: Hirsch Dynamics Holding AG, 8832 Wollerau (CH)
(72) Inventor: HIRSCH, Markus, 8832 Wollerau (CH)
(74) Representative: Torggler & Hofmann Patentanwälte - Innsbruck

(57) **Abstract**

A tool (1) for securing a bracket (2) of a fixation system for fixing a fracture in one or more bones to at least two rods or wires (3) which run in perpendicular directions, wherein said bracket (2) is secured to said at least two rods or wires (3) by snapping said at least two rods or wires into corresponding channels (4) formed on opposing surfaces of said bracket (2), wherein said tool (1) comprises at least a first part (5) and a second part (6) being movable relative to each other such that a reception space (7) is formed or can be formed between said first part (5) and said second part (6), inside which said bracket (2) can be positioned while in a state of being placed between said at least two perpendicularly running rods or wires (3), and a bracket (2) positioned in said reception space (7) is snapped onto said at least two rods or wires (3) by moving said first part (5) relative to said second part (6).

## Description

The invention concerns a tool for securing a bracket of a fixation system for fixing a fracture in one or more bones to at least two rods or wires which run in perpendicular directions, wherein said bracket is secured to said at least two rods or wires by snapping said at least two rods or wires into corresponding channels formed on opposing surfaces of said bracket, a fixation system comprising at least one such tool and a method for securing a bracket of a fixation system for fixing a fracture in one or more bones, outside of a body, to at least two rods or wires which run in perpendicular direction utilizing at least one such tool.

EP 3 422 971 B1 discloses a fixation system having rods and wires and brackets as described above.

It is an object of the invention to facilitate use of a fixation system of this type.

This object is achieved by a tool having the features of claim 1 and a fixation system and a method utilizing such a tool. Preferred embodiments of the invention are described in dependent claims. Such a tool allows easy and precise securing of the brackets to the orthogonally arranged wires or rods.

A preferred embodiment is described referring to the enclosed figures.
Figures 1 - 6 show application of a first embodiment of the tool for fixing one of a plurality of brackets to two perpendicularly running wires or rods.
Figures 7a, 7b show a modification of the first embodiment.
Figures 8 to 10 show application of a second embodiment of the tool for fixing one of a plurality of brackets to two perpendicularly running wires or rods.

As shown in EP 3 422 971 B1, a fixation system for fixating a fracture in one or more bones, comprises:
- a first plurality of wires or rods 3 adapted to be placed through said one or more bones to be fixed and extending out of a body containing said bones
- a second plurality of wires or rods 3 adapted to be placed in a perpendicular direction relative to said wires or rods 3 of said first plurality
- a plurality of brackets 2 to be secured to said pluralities of rods or wires 3 by snapping each bracket 2 to at least one rod or wire 3 of said first plurality and to at least one rod or wire 3 of said second plurality, said brackets 2 being provided with channels formed on opposing surfaces of a bracket 2 and running along perpendicular directions

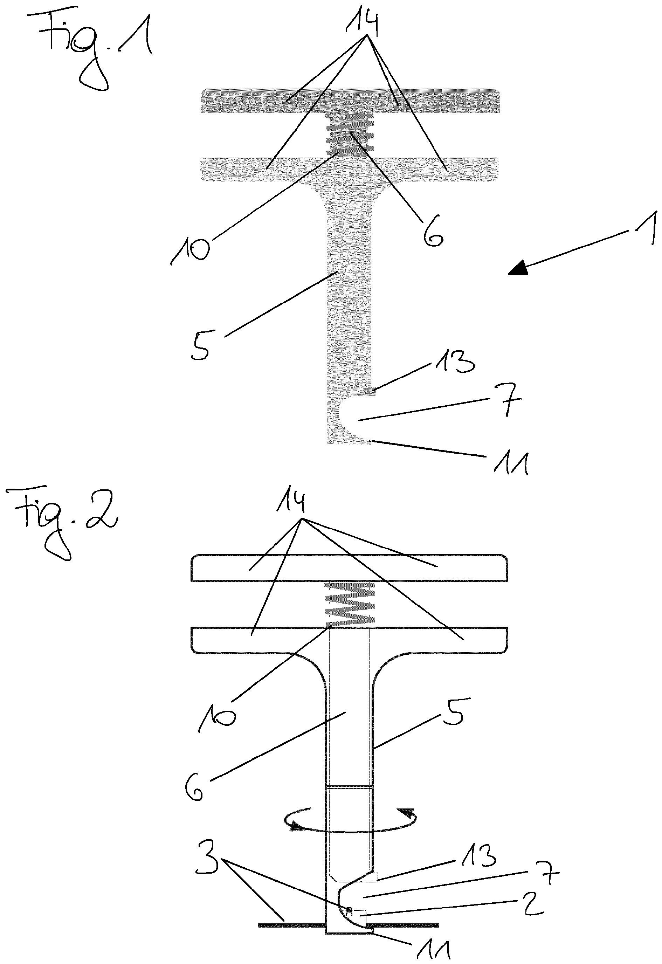

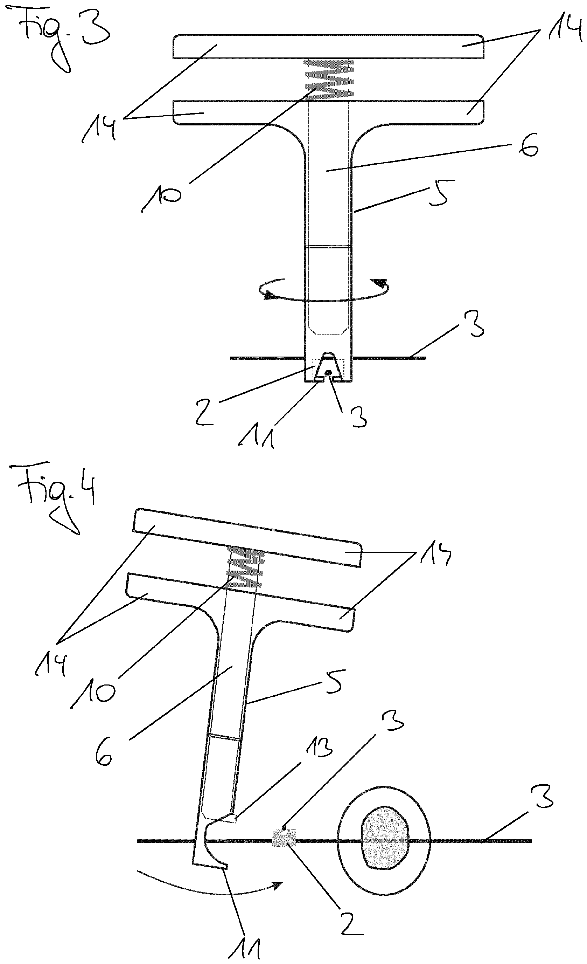

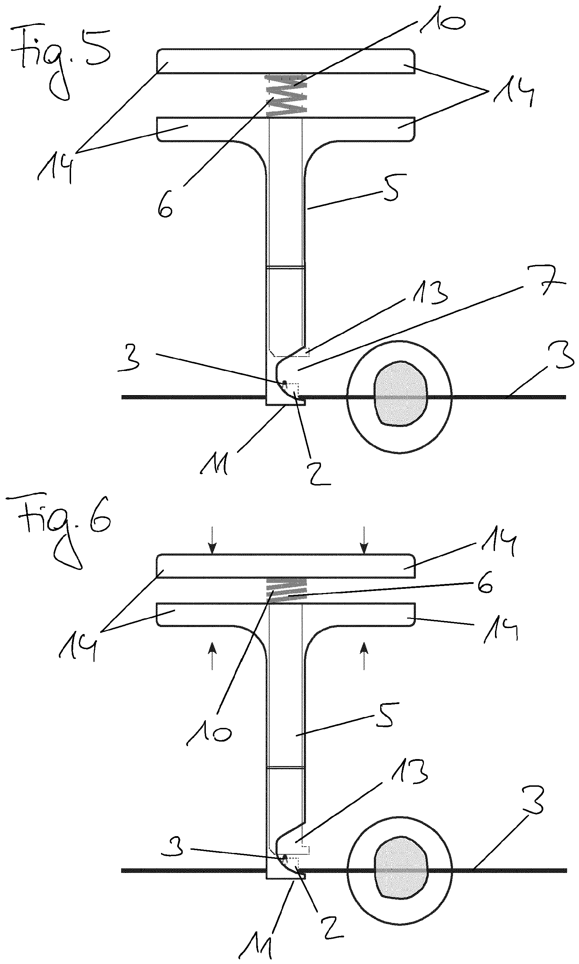

In the first embodiment of Figures 1 to 6, a first part 5 of the tool 1 has a sub-part in the general shape of a pipe having a central channel running through at least part of the length of the first part 5. A sub-part of a second part 6 of the tool 1 is in the general shape of a plunger 1 and is arranged movably inside the channel of the first part 5. Both, the first part 5 and the second part 6, have handles 14 (here in the form of flanges) which can be gripped by a user of the tool 1 (human or machine) to exert a force against an elastic force of an elastic member 10 (here: a spring or coil, alternatively: a block of an elastomer) arranged between the first part 5 and the second part 6 such that the sub-part of the second part 6 in the general shape of a plunger is moved along the channel of the first part 5 in a way that the handles 14 get closer to each other.

A reception space 7 is formed between a contacting surface 11 of said first part 5 and an end 13 of the plunger-shaped sub-part of the second part 6. As shown, a bracket 2 can be positioned in the reception space 7 such that it contacts the contacting surface while being in a state of being placed between two perpendicularly running rods or wires 3, because the reception space 7 has at least a first opening 8 and a second opening 9, the directions of which are arranged perpendicularly to each other to allow the perpendicular rods or wires 3 to pass through the reception space 7 when the tool 1 is arranged such that the bracket 2 is positioned in the reception space 7.

In the shown embodiment, the elastic member 10 serves to keep the reception space 7 open to allow the tool 1 to be arranged such that the bracket 2 is positioned in the reception space 7. In other words, the elastic member 10 is configured to hold the first part 5 and the second part 6 in a position such that a reception space 7 suitable for a bracket 2 to be arranged therein is formed. The elastic member 10 is loaded by moving the handles 14 nearer to each other.

By exerting a force on the handles 14 against the elastic force of the elastic member 10 the end 13 of said plunger-shaped part of the second part 6 is pressed against a first surface of the bracket 2 and the wire or rod 3 arranged thereon and a contacting surface 11 of the first part 5 is pressed against a second surface of the bracket 2 and the wire or rod 3 arranged thereon until both wires or rods 3 are snapped into corresponding channels formed on the first and second surface of the bracket 2.

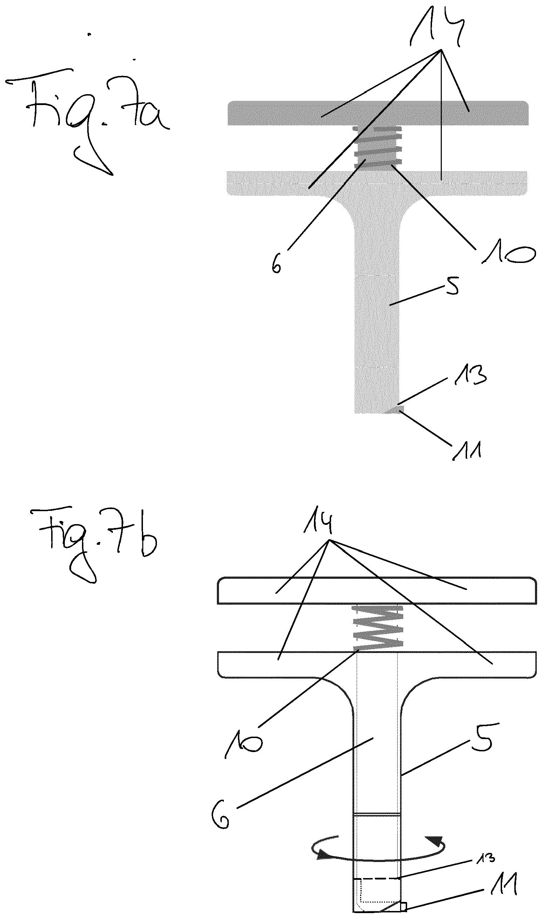

In an alternative embodiment, shown in Figures 7a and 7b, no reception space 7 is formed when the handles 14 are farthest from each other (as shown) and the reception space 7 is configured to receive a bracket 2 when the handles 14 are nearest to each other (not shown) and the elastic element 10 has been loaded by moving the handles 14 nearer to each other to exert a force on the at least two wires or rods 3 and the bracket 2 when the handles 14 are released in order to snap both wires or rods 3 into corresponding channels formed on the first and second surface of the bracket 2 by unloading the elastic member 10. In other words, the elastic member 10 is configured to reduce a size of the reception space 7 such that a bracket 2 positioned in the reception space 7 is snapped onto said at least two rods or wires 3 by the action of moving the first part 5 relative to said second part 6. This action can be supported by (such that additional force is to be provided by a user of the tool) or completely effected by the force of the elastic member 10.

Other than shown, such an alternative embodiment could also be obtained by replacing the compression spring shown in the first embodiment by a tension spring without any further modifications.

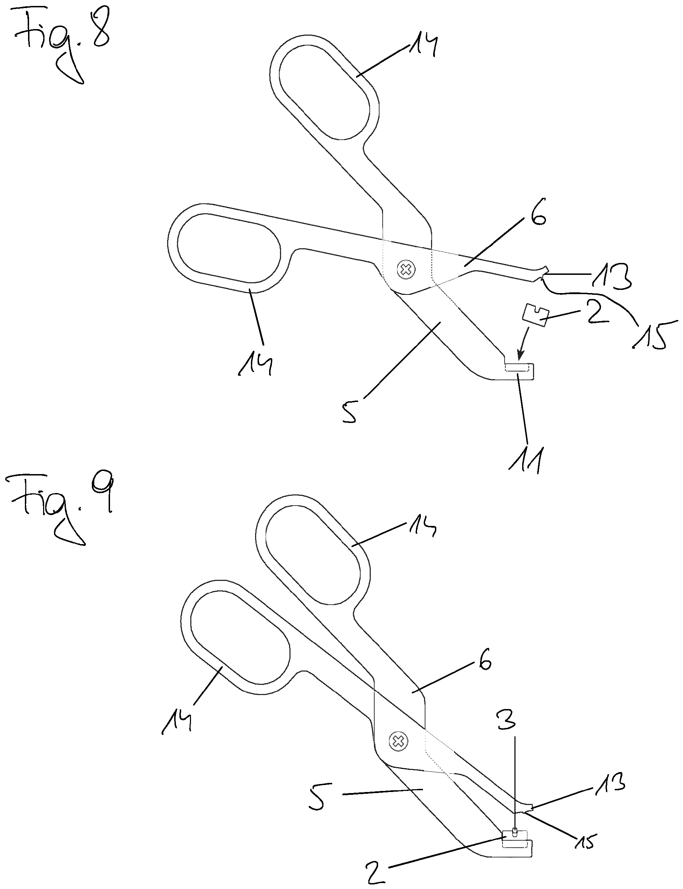

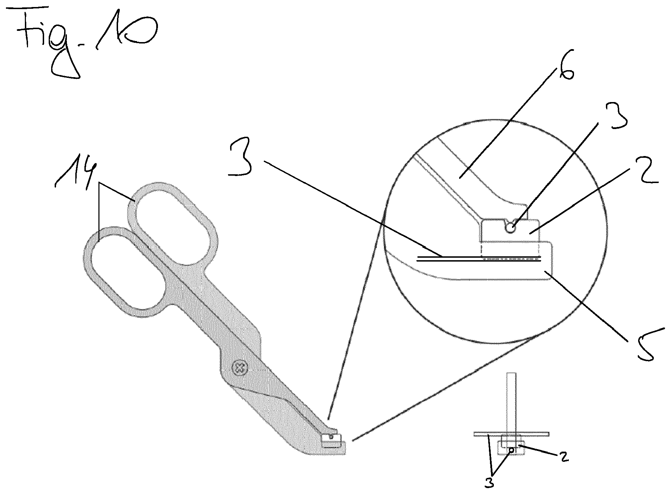

In the second embodiment shown in Figures 8 to 10, said first part 5 and said second part 6 are connected by a hinge 12 to form tongs such that said first part 5 and said second part 6 are movable relative to each other along an arch. In a plane arranged perpendicular to an axis of said hinge 12, said first part 5 and said second part 6 are offset such that the one wire or rod 3 running in said plane can pass the first part 5 or second part 6. As can be seen, the end 13 of the second part 6 can have a nose 15 to facilitate pressing the one wire or rod 3 into the channel formed on the bracket 2.

It is a preferred option, with respect to both embodiments, that said reception space 7 comprises a receptacle shaped such that said bracket 2 is rotationally locked relative to said receptacle when being placed (usually only part of said bracket 2 is received by said receptacle) in said receptacle. If this option is present, in particular with respect to the first embodiment or its modification, said receptacle can be arranged on said first part 5 or said second part 6 by way of a rotating joint which, preferably, has an axis arranged perpendicular to said directions of said at least one first opening 8 and at least one second opening 9.

It is to be noted that while in the first embodiment there is only one first opening 8 and only one second opening 9 since in the first embodiment said first part 5 partially encloses said reception space 7, in the second embodiment it could be said that there is an infinite number of first openings 8 and second openings 9 since in the second embodiment said reception space 7 is open in all directions (except, of course, where the material of the first part 5 and the second part 6 is present).

## Claims

1. A tool (1) for securing a bracket (2) of a fixation system for fixing a fracture in one or more bones to at least two rods or wires (3) which run in perpendicular directions, wherein said bracket (2) is secured to said at least two rods or wires (3) by snapping said at least two rods or wires into corresponding channels (4) formed on opposing surfaces of said bracket (2), **characterised in that** said tool (1) comprises at least a first part (5) and a second part (6) being movable relative to each other such that a reception space (7) is formed or can be formed between said first part (5) and said second part (6), inside which said bracket (2) can be positioned while in a state of being placed between said at least two perpendicularly running rods or wires (3), and a bracket (2) positioned in said reception space (7) is snapped onto said at least two rods or wires (3) by moving said first part (5) relative to said second part (6).

2. The tool of the preceding claim, wherein said first part (5) has a sub-part in the general shape of a pipe having a central channel running through at least part of a length of the first part (5) and a sub-part of the second part (6) is in the general shape of a plunger which is arranged movably along said channel of said first part (5) such that said first part (5) and said second part (6) are movable relative to each other such that a bracket (2) positioned in said reception space (7) is snapped onto said at least two rods or wires (3) by the action of moving said first part (5) relative to said second part (6).

3. The tool of claim 1, wherein said first part (5) and said second part (6) are connected by a hinge (12) to form tongs such that said first part (5) and said second part (6) are movable relative to each other along an arch such that a bracket (2) positioned in said reception space (7) is snapped onto said at least two rods or wires (3) by the action of moving said first part (5) relative to said second part (6).

4. The tool of at least one of the preceding claims, wherein said reception space (7) comprises a receptacle shaped such that said bracket (2) is rotationally locked when being placed in said receptacle.

5. The tool of the preceding claim, wherein said receptacle is arranged on said first part (5) or said second part (6) by way of a rotating joint which, preferably, has an axis arranged perpendicular to said at least two rods or wires (3) .

6. The tool of at least one of the preceding claims, wherein at least one elastic member (10) is arranged between said first part (5) and said second part (6), such that said first part (5) and said second part (6) are movable relative to each other against an elastic force of said at least one elastic member (10).

7. The tool of the preceding claim, wherein said at least one elastic member (10) is configured to hold said first part (5) and said second part (6) by elastic force in a position such that said reception space (7) is formed.

8. The tool of claim 6, wherein said at least one elastic member (10) is configured to reduce by elastic force a size of said reception space (7) such that a bracket (2) positioned in said reception space (7) is snapped onto said at least two rods or wires (3) by the action of moving said first part (5) relative to said second part (6).

9. The tool of one of claims 6 to 8, wherein said elastic member (10) is a spring or coil or a block of an elastomer.

10. The tool of at least one of the preceding claims, wherein said first part (5) and said second part (6) have at least one handle (14) which can be gripped by a user of said tool (1) to cause relative motion of said first part (5) and said second part (6).

11. The tool of at least one of the preceding claims, wherein said reception space (7) has at least a first opening (8) and a second opening (9), the directions of which are arranged perpendicularly to each other to allow said at least two rods or wires (3) to pass through said reception space (7) when said tool (1) is arranged such that said bracket (2) is positioned in said reception space (7).

12. A fixation system for fixating a fracture in one or more bones, comprising at least:
- a first plurality of wires or rods (3) adapted to be placed through said one or more bones to be fixed and extending out of a body containing said bones
- a second plurality of wires or rods (3) adapted to be placed in a perpendicular direction relative to said wires or rods (3) of said first plurality
- a plurality of brackets (2) to be secured to said pluralities of rods or wires (3) by snapping each bracket (2) to at least one rod or wire (3) of said first plurality and to at least one rod or wire (3) of said second plurality, said brackets (2) being provided with channels formed on opposing surfaces of a bracket (2) and running along perpendicular directions
- at least one tool (1) according to at least one of claims 1 to 11.

13. A method for securing a bracket (2) of a fixation system, in particular according to claim 12, for fixing a fracture in one or more bones, outside of a body, to at least two rods or wires (3) which run in perpendicular directions, said method utilizing a tool (1) according to at least one of claims 1 to 11.
